# EUROPÄISCHE PATENTANMELDUNG

(11) **EP 1 300 136 A2**
(43) Veröffentlichungstag der Anmeldung: **09.04.2003**
(21) Anmeldenummer: 02021424.3
(22) Anmeldetag: 25.09.2002
(51) Int. Cl.: A61K 7/135

(54) **Farbveränderndes Verfahren zur Haarbehandlung auf Basis von Dithionitsalzen**

(30) Priorität: 04.10.2001 DE 10148848; 26.10.2001 DE 10152940
(71) Anmelder: Henkel Kommanditgesellschaft auf Aktien, 40589 Düsseldorf-Holthausen (DE)
(72) Erfinder: Ehlers, Heide, 41470 Neuss (DE); Höffkes, Horst, Dr., 40595 Düsseldorf (DE); Fiedler, Sigrid, 40667 Meerbusch (DE)

(57) **Zusammenfassung**

Die vorliegende Anmeldung betrifft ein Verfahren zur Behandlung keratinischer Fasern, bei dem in einem ersten Schritt die Fasern in üblicher Weise gefärbt werden und in einem zweiten Schritt die Fasern mit einem Mittel (M), enthaltend ein Dithionitsalz und mindestens ein anionisches Tensid behandelt werden. Das erfindungsgemäße Verfahren ermöglicht einen Farbabzug bei gleichzeitiger Schonung der Fasern.

## Beschreibung

Die vorliegende Erfindung betrifft ein spezielles farbveränderndes Verfahren zur Haarbehandlung auf Basis von Dithionitsalzen, entsprechende Mittel, enthaltend diese Dithionitsalze sowie die Verwendung dieser Mittel zur Beschleunigung des Auswaschens von Färbungen und zur Veränderung der Farbnuance.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel, wie beispielsweise Wasserstoffperoxid-Lösungen. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Färbemittel auf der Basis direktziehender Farbstoffe werden häufig für temporäre Färbungen eingesetzt. Bei den direktziehenden Farbstoffen handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna.

Bei all diesen Färbeprozessen kann es vorkommen, dass die Färbung aus verschiedenen Gründen ganz oder teilweise wieder rückgängig gemacht werden soll. Eine teilweise Entfernung der Färbung bietet sich beispielsweise an, wenn das Färbeergebnis auf den Fasern dunkler ausfällt als gewünscht. Es ist daher eine Aufgabe der vorliegenden Erfindung ein Verfahren sowie entsprechend Mittel bereitzustellen, mit denen eine gezielte anschließende Nachnuancierung gefärbter Fasern möglich ist, ohne die Faser selbst unnötig anzugreifen.

Andererseits kann auch eine vollständige Entfernung der Färbung in manchen Fällen gewünscht sein. So ist es beispielsweise denkbar, dass die Haare für einen konkreten Anlass in einer bestimmten Nuance gefärbt oder getönt werden sollen und nach einigen Tagen die ursprüngliche Farbe wieder zurückgewonnen werden soll. Daher ist es eine weitere Aufgabe der vorliegenden Erfindung ein Verfahren und entsprechende Mittel zur Verfügung zu stellen, die einen vollständigen Farbabzug ermöglichen ohne die Fasern selbst unnötig zu schädigen.

Mittel und Verfahren zum Farbabzug sind bereits literaturbekannt. Üblicherweise werden Färbungen oxidativ, beispielsweise mithilfe eines üblichen Blondierverfahrens wieder rückgängig gemacht. Bei diesem Prozess können die Fasern aber durch den Einsatz starker Oxidationsmittel geschädigt werden. Ferner wurden auch reduktive Prozesse zum Farbabzug bereits beschrieben. So offenbart beispielsweise das deutsche Patent DE-197 37 987-C1 Mittel zur Entfärbung von gefärbten Haaren, die mindestens einen Alkohol sowie ein schwefelhaltiges Reduktionsmittel enthalten. Diese Methoden des Standes der Technik können allerdings hinsichtlich der erzielten Wirkung, insbesondere der Gleichmäßigkeit der resultierenden Färbung, noch nicht vollständig überzeugen.

Es wurde nun überraschenderweise gefunden, dass Haarbehandlungsmittel auf Basis von Dithionitsalzen und Tensiden die an farbverändemde Mittel gestellten Anforderungen in einem hohen Maße erfüllen und gleichzeitig die Fasern schonen.

Ein erster Gegenstand der vorliegenden Erfindung ist daher ein Verfahren zur Behandlung keratinischer Fasern, bei dem in einem ersten Schritt die Fasern in üblicher Weise gefärbt werden und in einem zweiten Schritt die Fasern mit einem Mittel (M), enthaltend ein Dithionitsalz und mindestens ein Tensid, behandelt werden.

Unter Keratinfasern sind dabei Pelze, Wolle, Federn und insbesondere menschliche Haare zu verstehen.

Der Begriff "in üblicher Weise gefärbt werden" im ersten Schritt des erfindungsgemäßen Verfahrens umfasst dabei alle dem Fachmann bekannten Verfahren, bei denen auf das Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Die Zusammensetzung des herkömmlichen Färbemittels unterliegt keinen prinzipiellen Einschränkungen.

Als Farbstoff(vorprodukt)e können
- Oxidationsfarbstoffvorprodukte vom Entwickler- und Kuppler-Typ,
- synthetische direktziehende Farbstoffe und
- natürliche Farbstoffe, wie beispielsweise Henna,
sowie Mischungen von Vertretern einer oder mehrerer dieser Gruppen eingesetzt werden.

In einer ersten Ausführungsform des erfmdungsgemäßen Verfahrens ist der erste Schritt eine oxidative Färbung. Im Rahmen dieser Ausführungsform werden Oxidationsfarbstoffvorprodukte vom Entwickler- und Kuppler-Typ unter Ausbildung der eigentlichen Farbstoffe oxidiert.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2'-hydroxyethyl)-p-phenylendiamin, 2-(2',5'-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4'-aminophenyl)-diazacycloheptan, 1,3-Bis(N(2'-hydroxyethyl)-N(4'-aminophenylamino))-2-propanol, 4-Amino-2-(2'-hydroxyethoxy)-phenol, 1,10-Bis-(2',5'-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol.

Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Di-(β-hydroxyethylamino)-toluol und 2,6-Dihydroxy-3,4-dimethylpyridin.

Ferner können die Mittel zur oxidativen Haarfärbung zur weiteren Nuancierung direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In einer zweiten bevorzugten Ausführungsform dieses Gegenstandes der vorliegenden Erfindung ist der erste Schritt des erfindungsgemäßen Verfahrens eine Färbung auf Basis von direktziehenden Farbstoffen, d.h. es wird auf den Einsatz von Oxidationsfarbstoffvorprodukten verzichtet. Hinsichtlich der bevorzugten direktziehenden Farbstoffe sei an dieser Stelle explizit auf die oben dargelegten Ausführungen verwiesen.

Zur weiteren Nuancierung können in beiden bevorzugten Ausführungsformen neben den Farbstoff(vorprodukt)en auch in der Natur vorkommende direktziehende Farbstoffe, wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel, eingesetzt werden.

Es ist nicht erforderlich, dass die Oxidationsfarbstoffvorprodukte oder die direktziehenden Farbstoffe jeweils einheitliche Verbindungen darstellen. Vielmehr können in den Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Sowohl die Oxidationsfarbstoffvorprodukte als auch die direktziehenden Farbstoffe sind in den Mitteln bevorzugt in Mengen von 0,01 bis 20 Gew.-%, vorzugsweise 0,5 bis 5 Gew.-%, jeweils bezogen auf das gesamte Mittel, enthalten.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkalioder Erdalkalihydroxide, Ammoniak oder organische Amine. Bevorzugte Alkalisierungsmittel sind Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel.

So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, dass dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Der zweite Schritt des erfindungsgemäßen Verfahrens ist die Behandlung der keratinischen Fasern mit einem Mittel (M), enthaltend mindestens ein Dithionitsalz und mindestens ein Tensid.

Erfindungsgemäß sind prinzipiell alle physiologisch verträglichen Salze des Dithionits, wie beispielsweise Natriumdithionit, Kaliumdithionit und Ammoniumdithionit geeignet. Ein erfindungsgemäß besonders bevorzugtes Dithionitsalz ist Natriumdithionit.

Das in dem erfindungsgemäßen Verfahren verwendete Mittel (M) enthält bevorzugterweise 0,5 bis 20 Gew.-% eines Dithionitsalzes, Mengen von 2 bis 10 Gew.-% sind besonders bevorzugt. Es kann aber auch erfindungsgemäß bevorzugt sein, den Gehalt an Dithionit entsprechend dem Zustand der zu behandelnden Haare zu wählen. So kann es bei bereits vorgeschädigten Haaren bevorzugt sein, 0,5 bis 3 Gew.-% eines Dithionitsalzes einzusetzen, während bei gesundem Haar vorzugsweise ein Dithionitsalzgehalt von 5 bis 10Gew.-% eingesetzt wird.

Die erfindungsgemäßen Mittel (M) enthalten mindestens ein Tensid, wobei prinzipiell sowohl anionische, nichtionische als auch zwitterionische, ampholytische und kationische Tenside geeignet sind. Erfindungsgemäß besonders bevorzugt sind die anionischen Tenside. In vielen Fällen hat es sich aber auch als vorteilhaft erwiesen, dass die Mittel ein oder mehrere weitere Tenside, insbesondere ausgewählt aus den zwitterionischen und nichtionischen Tensiden enthalten.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosrn.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quatemäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Es hat sich als erfindungsgemäß bevorzugt erwiesen, wenn das Mittel M im Rahmen des Verfahrens eine ausreichende Viskosität aufweist, so dass es sich auf den Fasern gut verteilen läßt ohne leicht zu verlaufen. Die Viskositäten können prinzipiell mit allen herkömmlichen Methoden eingestellt werden. So hat es sich beispielsweise als vorteilhaft erwiesen, die Mittel mithilfe von
- organischen, natürlichen Verdickungsmitteln, wie beipielsweise Agar-Agar, Xanthan-Gum, Carrageen, Traganth, Gummi arabicum, Alginate, Pektine, Polyosen, GuarMehl, Johannisbrotbaumkernmehl, Leinsamengummen, Stärke-Fraktionen und Derivate wie Amylose, Amylopektin und Dextrine, Dextrane, Gelatine oder Casein,
- organischen, abgewandelten Naturstoffen, wie beispielsweise Kemmehlether oder Cellulose-Derivate, wie beispielsweise Methylcellulose, Hydroxyalkylcellulose, insbesondere Hydroxyethyl- und -propylcellulose, Carboxymethylcellulose sowie Celluloseester,
- organische, vollsynthetische Verdickungsmittel, wie beispielsweise Polyacryl- u. Polymethacryl-Verbindungen, Vinylpolymere, wie beispielsweise Polyvinylpyrrolidon oder Poylvinylakohol, Polycarbonsäuren, Polyether, Polyimine oder Polyamide, oder
- anorganischen Verdickungsmitteln, wie beispielsweise Polykieselsäuren, Tonmineralien wie Montmorillonite, Bentonit, Zeolithe oder Kieselsäuren
zu verdicken. Die erfindungsgemäß verdickten Mittel zeichnen sich durch deutlich gleichmäßigere Färbeergebnisse aus, als dies bei vergleichbaren dünnflüssigen Mitteln der Fall ist.

In vielen Fällen hat es sich aber als ausreichend erwiesen, die Mittel mithilfe eine Fettalkoholsystems zu verdicken.

Die Mittel (M) des erfindungsgemäßen Verfahrens weisen bevorzugt einen pH-Wert von 4 bis 12, insbesondere von 6 bis 10, auf. Falls das Mittel M in einem erfindungsgemäßen Verfahren zum Einsatz kommt, bei dem der erste Schritt eine oxidative Haarfärbung darstellt, weist das Mittel bevorzugt einen pH-Wert von 6,5 bis 12 auf, eine Anwendung im alkalischen Bereich ist ganz besonders bevozugt.

Die Einwirkzeit des Mittels (M) im Rahmen des erfindungsgemäßen Verfahrens beträgt bevorzugterweise zwischen 5 und 60 Minuten, besonders bevorzugt sind Einwirkzeiten zwischen 15 und 45 Minuten.

Zur Erhöhung der Lagerstabilität kann es gemäß einer Ausführungsform der vorliegenden Erfindung bevorzugt sein, das Mittel (M) als Zweikomponentensystem zu formulieren, dessen Einzelzubereitungen unmittelbar vor der Anwendung vermischt werden.

Im Rahmen dieser Ausführungsform kann das Mittel (M) unmittelbar vor der Anwendung durch Mischen einer ersten, wässrigen oder wässrig-alkoholischen Zubereitung (I) mit einer zweiten wasserfreien Zubereitung (II), enthaltend mindestens ein Dithionitsalz, erhalten werden, wobei mindestens eine der Zubereitungen (I) oder (II) mindestens ein Tensid enthält.

Als wasserfrei im Sinne der vorliegenden Erfindung werden Zubereitungen mit einem Wassergehalt unter 1 Gew.-% verstanden.

In einer bevorzugten Ausführungsform ist die wasserfreie Zubereitung (II) pulverförmig. Falls das Dithionitsalz in einer pulverförmigen Zubereitung formuliert ist, kann erfmdungsgemäß bevorzugt sein, diese Zubereitung vor der Mischung mit der wässrigen oder wässrig-alkoholischen Zubereitung (I) in etwas Wasser vorzulösen, da hierdurch eine
schnelle und verbesserte Homogenisierung erreicht wird.

In einer ebenfalls bevorzugten Ausführungsform kann die wasserfreie Zubereitung (II) auch als wasserfreie Paste vorliegen. Obwohl die vorliegenden Erfindung hinsichtlich der geeigneten Pastengrundlage in keiner Weise eingeschränkt ist, hat es sich als bevorzugt erwiesen, wenn die Grundlage eine Kombination aus wasserunlöslichen flüssigen Ölkomponenten, nichtionischen Emulgatoren mit einem HLB-Wert von 5 oder mehr, Verdickungsmitteln, bevorzugterweise ausgewählt aus einer Gruppe, bestehend aus C₁₆-C₁₈-Fettsäurepartialestern von C₂-C₆-Polyolen mit 2 bis 6 Hydroxygruppen und C₁₆-C₁₈-Fettsäure-Metallseifen sowie gegebenenfalls wasserlöslichen Polyolen oder Polyethylenglycolen enthält. Ferner hat es sich als vorteilhaft erwiesen, wenn die Pastengrundlagen darüber hinaus noch Verdünnungsmittel, wie beispielsweise Alkylencarbonate, insbesondere Propylencarbonat, enthalten.

Unter wässrig-alkoholischen Zubereitungen sind im Sinne der vorliegenden Erfindung wässrige Lösungen enthaltend 3 bis 70 Gew.-% eines C₁-C₄-Alkohols, insbesondere Ethanol oder Isopropanol, zu verstehen.

Hinsichtlich des zeitlichen Ablaufs des erfindungsgemäßen Verfahrens sind prinzipiell keine Grenzen gesetzt. Vielmehr wird der zeitliche Ablauf stark durch den gewünschten Effekt der Haarbehandlung dominiert. Ist es beispielsweise beabsichtigt, die Nuance der erzielten Färbung zu optimieren, so wird der zweite Schritt des erfindungsgemäßen Verfahrens unmittelbar im Anschluss an den Färbeprozess stattfinden. Kommt das erfindungsgemäße Verfahren zum Einsatz, um eine erzielte Färbung wieder vollständig zu entfernen, so kann der zweite Verfahrensschritt bei Missfallen der erzielten Färbung ebenfalls unmittelbar nach dem Färbeprozess stattfinden. Der zweite Verfahrensschritt kann aber erfindungsgemäß auch zu einem späteren Zeitpunkt, beispielsweise nach einigen Tagen, durchgerührt werden, wenn die Haarfärbung nicht länger erwünscht ist. In diesen Fällen wird mithilfe des erfindungsgemäßen Verfahrens gezielt das Auswaschen der erzielten Färbung beschleunigt.

Im Rahmen der Versuche der Anmelderin hat es sich gezeigt, dass es erfindungsgemäß bevorzugt sein kann, die Fasern während der Einwirkzeit des Mittels (M), enthaltend das Dithionitsalz und mindestens ein Tensid, von der unmittelbaren Luftzufuhr abzuschirmen. Dies kann beispielsweise verwirklicht werden, indem man die Fasern unmittelbar nach der Applikation des Mittels (M) mit einer Folie, insbesondere mit einer Aluminiumfolie, umwickelt.

Weiterhin hat es sich als vorteilhaft erwiesen, die Fasern nach dem erfindungsgemäßen Behandlungsverfahren mit einer sauer eingestellten Lösung einer physiologisch verträglichen anorganischen und/oder organischen Säure zu behandeln. Diese Spülung weist bevorzugterweise einen pH-Wert zwischen 2 und 6, insbesondere zwischen 3 und 4, auf. Die Anwendung einer derartigen sauren Spülung verbessert einerseits die Faserstruktur und damit den Glanz und den Pflegezustand der Fasern. Andererseits wird aber auch das Ergebnis der Farbveränderung durch ein derartiges saures Nachspülen verbessert.

In einer bevorzugten Ausführungsform dieser Erfindung wird das Mittel (M) mit einer derartigen sauren Spülung aus den Fasern gewaschen. Es hat sich gezeigt, dass dann das Färbeergebnis gleichmäßiger ist und eine bessere Entfärbung erzielt werden kann.

In einer weiteren bevorzugten Ausführungsform der vorliegenden Erfindung kann es bevorzugt sein, wenn die Fasen im Rahmen der Nachbehandlung einer leichten Fixierung unterzogen werden. Die Art der Fixierung unterliegt erfindungsgemäß keinerlei Beschränkungen. Typische chemische Oxidationsmittel, wie beispielsweise Natriumbromat, Kaliumbromat oder Wasserstoffperoxid können ebenso eingesetzt werden wie enzymatische Fixiermittel, wie beispielsweise Peroxidasen. Es kann dabei erfindungsgemäß besonders bevorzugt sein, wenn die sauer eingestellten Nachbehandlungsmittel weiterhin Wasserstoffperoxid enthalten.

Ein zweiter Gegenstand der vorliegenden Erfindung sind Mittel zur Farbveränderung gefärbter keratinischer Fasern, die unmittelbar vor der Anwendung durch Mischen einer ersten, wässrigen oder wässrig-alkoholischen Zubereitung (I) mit einer zweiten wasserfreien Zubereitung (II), enthaltend mindestens ein Dithionitsalz, erhalten wird, wobei mindestens eine der Zubereitungen (I) oder (II) mindestens ein Tensid enthält.

Hinsichtlich der Definition der verwendeten Begriffe sei an dieser Stelle explizit auf die obigen Ausführungen verwiesen.

Auch im Rahmen dieses Erfindungsgegenstandes ist es besonders bevorzugt, wenn mindestens eine der Zubereitungen (I) oder (II) mindestens ein anionisches Tensid enthält.

Obwohl beide Zubereitungen des erfmdungsgemäßen zweiteiligen Mittels prinzipiell das Tensid enthalten können, ist es besonders bevorzugt, wenn das Tensid in der Zubereitung (I) enthalten ist.

Weiterhin enthalten die erfindungsgemäßen Mittel bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öloder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle. Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-nundecylether, Di-n-dodecylether, n-Hexyl-n-octylether, n-Octyl-n-decylether, n-Decyl-nundecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-noctylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-diisostearat und Neopentylglykoldi-capylat erfindungsgemäß verwendbare Ölkomponenten dar, ebenso komplexe Ester wie z. B. das Diacetyl- glycerinmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Fettalkohole sind erfindungsgemäß besonders bevorzugte Ölkomponenten. Obwohl die Ölkomponente auch in der Zubereitung (II) enthalten sein kann, sind solche Mittel erfindungsgemäß bevorzugt, bei denen in der Zubereitung (I) mindestens eine Ölkomponente enthalten ist.

In einer bevorzugten Ausführungsform der vorliegenden Erfindung enthalten die erfindungsgemäßen Mittel neben dem Dithionitsalz mindestens ein weiteres Reduktionsmittel. Beispiele für geeignete Reduktionsmittel sind Thioglykolsäure, Thiomilchsäure, Thioglycerol, Mercaptopropionsäure, Natriumhydrogensulfit, Ammnoniumhydrogensulfit, Cysteamin, Dithiothreitol, Thioäpfelsäure oder α-Mercaptoethylsulfonsäure. Ferner sind die Sulfit- und Disulfitsalze bevorzugte Reduktionsmittel. Ein besonders bevorzugtes Reduktionsmittel ist Natriumdisulfit. Es kann erfindungsgemäß bevorzugt sein, dass das weitere Reduktionsmittel in der Zubereitung (II) enthalten ist.

Die erfindungsgemäßen Mittel enthalten das weitere Reduktionsmittel bevorzugt in Mengen von 0,2 bis 5,0 Gew.-%, insbesondere in Mengen von 0,5 bis 2,0 Gew.-% .

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quatemierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen, sowie Substanzen zur Stabilisierung des eingestellten pH-Wertes, wie beispielsweise anorganische Sulfatsalze,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein dritter Gegenstand der vorliegenden Erfindung ist die Verwendung einer Anwendungszubereitung, enthaltend mindestens ein Dithionitsalz und mindestens ein Tensid, zur Beschleunigung des Auswaschens von Färbungen keratinischer Fasern.

Ein vierter Gegenstand der vorliegenden Erfindung ist die Verwendung einer Anwendungszubereitung, enthaltend mindestens ein Dithionitsalz und mindestens ein Tensid, zur Nachnuancierung von Färbungen keratinischer Fasern.

Auch im Rahmen dieser Gegenstände der vorliegenden Erfindung hat sich der Einsatz mindestens eines anionischen Tensides als besonders bevorzugt erwiesen.

Die folgenden Beispiele sollen den Gegenstand der vorliegenden Erfindung näher erläutern ohne in jedoch zu beschränken.

### Beispiele

Die Angaben in den Beispielen sind, sofern nichts anderes vermerkt ist, in Gewichtsprozent.

### 1. Zusammensetzung des farbverändernden Mittels

| Zusammensetzung (Ia) | |
|---|---|
| Texapon® NSO¹ | 8,0 |
| Lorol®, technisch² | 3,5 |
| Hydrenol® D³ | 4,5 |
| Eumulgin® O5⁴ | 2,0 |
| AMPD⁵ | 0,6 |
| Wasser | ad 100 |
| Der pH-Wert dieser Zusammensetzung beträgt 9,3. | |

| | |
|---|---|
| ¹ Laurylethersulfat, Natriumsalz (ca. 27,5% Aktivsubstanz; INCI-Bezeichnung: Sodium Laureth Sulfate) (HENKEL) | |
| ² C₁₂₋₁₈-Fettalkohol (INCI-Bezeichnung: Coconut alcohol) (HENKEL) | |
| ³ C₁₆₋₁₈-Fettalkohol (INCI-Bezeichnung: Cetearyl alcohol) (HENKEL) | |
| ⁴ Oleyl-Cetylalkohol mit ca. 5 EO-Einheiten (INCI-Bezeichnung: Oleth-5) (HENKEL) | |
| ⁵ 2-Amino-2-methyl-1,3-propandiol (INCI-Bezeichnung: Aminomethyl Propanediol) | |

| Zusammensetzung (Ib) | |
|---|---|
| Texapon® NSO | 8,0 |
| Lorol®, technisch | 3,5 |
| Hydrenol® D | 4,5 |
| Eumulgin® O5 | 2,0 |
| Citronensäure | 0,3 |
| Phenoxyethanol | 0,5 |
| Methylparaben | 0,3 |
| Propylparaben | 0,3 |
| Der pH-Wert dieser Zusammensetzung beträgt 6,8. | |

| Zusammensetzung (II) | |
|---|---|
| Natriumdithionit | 95 |
| Natriumdisulfit | 5 |

### 2. Zusammensetzung der Färbemittel

Es wurden die folgenden Färbemittel hergestellt:

| Färbemittel A | |
|---|---|
| Akypo® RLM 45 N⁶ | 3,0 |
| Hydrenol® D | 2,0 |
| Lorol, techn. | 2,5 |
| Aminol® A 15⁷ | 0,5 |
| Paridol® M⁸ | 0,3 |
| Paridol® P⁹ | 0,2 |
| Phenoxyethanol | 0,8 |
| Weinsäure | 0,2 |
| HC Violet¹⁰ | 0,25 |
| HC Red No. 3¹¹ | 0,45 |
| HC Blue No. 2¹² | 0,3 |
| Lowadene® violet 1¹³ | 0,3 |
| Carbopol® ETD 2001¹⁴ | 0,15 |
| AMP¹⁵ | 0,8 |
| Parfüm | 0,1 |
| Merquat® 280¹⁶ | 0,5 |
| d-Panthenol | 0,2 |
| d,1-α-Tocopherolacetat | 0,2 |
| Wasser | ad 100 |

| | |
|---|---|
| ⁶ C₁₂₋₁₄-Fettalkohol-4.5-EO-Essigsäure-Natrium-Salz (ca. 80 bis 84% Aktivsubstanzgehalt; INCI-Bezeichnung: Sodium Laureth-6 Carboxylate) (Chem-Y) | |
| ⁷ Alkylethercarboxylatmonoethanolamid (ca. 97 bis 100% Aktivsubstanzgehalt, INCI-Bezeichnung: Trideceth-2 Carboxamide Mea) (Chem-Y) | |
| ⁸ 4-Hydroxybenzoesäuremethylester (INCI-Bezeichnung: Methylparaben) (Naarden) | |
| ⁹ 4-Hydroxybenzoesäurepropylester (INCI-Bezeichnung: Propylparaben) (Naarden) | |
| ¹⁰ 1,4-Bis[(2'-hydroxyethyl)-amino]-2-nitrobenzol | |
| ¹¹ N'(2-Hydroxyethylamino)-2-nitro-4-aminobenzol | |
| ¹² N,N,N'-Tris(2-hydroxyethyl)-2-nitro-1,4-phenylendiamin | |
| ¹³ 1,4-Diaminoanthrachinon | |
| ¹⁴ Polyacrylsäure (ca. 98 bis 100% Aktivsubstanz; INCI-Bezeichnung: Carbomer) (Goodrich) | |
| ¹⁵ 2-Amino-2-methylpropanol (ca. 95% Aktivsubstanzgehalt; INCI-Bezeichnung: Aminomethyl Propanol) | |
| ¹⁶ Dimethyldiallylammoniumchlorid-Acrylsäure-Copolymer (ca. 35% Aktivsubstanzgehalt; INCI-Bezeichnung: Polyquaternium-22) (Chemviron) | |

| Färbemittel B | |
|---|---|
| Akypo® RLM 45 N | 3,0 |
| Hydrenol® D | 2,0 |
| Lorol, techn. | 2,5 |
| Aminol® A 15 | 0,5 |
| Paridol® M | 0,3 |
| Paridol® P | 0,2 |
| Phenoxyethanol | 0,8 |
| Weinsäure | 0,2 |
| HC Red No. 1¹⁷ | 0,2 |
| HC Orange No. 1¹⁸ | 0,6 |
| HC Red BN¹⁹ | 0,2 |
| HC Red No. 3 | 0,5 |
| Carbopol® ETD 2001 | 0,15 |
| AMP | 0,75 |
| Parfüm | 0,1 |
| Merquat® 280 | 0,5 |
| d-Panthenol | 0,2 |
| d,1-α-Tocopherolacetat | 0,2 |
| Wasser | ad 100 |

| | |
|---|---|
| ¹⁷ 4-Amino-2-nitrodiphenylamin | |
| ¹⁸ 2-Nitro-4'-hydroxydiphenylamin | |
| ¹⁹ 4-[(3'-Hydroxypropyl)amino]-3-nitrophenol | |

### 3. Zusammensetzung des Nachbehandlungsmittels

Es wurde das folgende Nachbehandlungsmittel hergestellt.

| | |
|---|---|
| Rewoquat® W 75 PG²⁰ | 2,5 |
| Dehyquart® F75²¹ | 1,0 |
| Cetyl-Stearylalkohol | 2,5 |
| Tego Amid® S 18²² | 0,8 |
| Glyzerinmonostearat | 0,8 |
| Isopropylmyristat | 1,0 |
| p-Hydroxybenzoesäuremethylester | 0,2 |
| Citronensäure (mind. 46%ig) | 0,9 |
| Gluadin® W 20²³ | 1,0 |
| Dow Corning® 1401 Fluid²⁴ | 2,0 |
| Mandelproteinhydrolysat | 1,0 |
| Panthenol 75 L | 0,2 |
| Phenoxyethanol | 0,4 |
| Parfüm | 0,3 |
| Wasser | ad 100 |

| | |
|---|---|
| ²⁰ 1-Methyl-2-nortalgalkyl-3-talgfettsäureamidoethylimidazolinium-methosulfat in-Propylenglykol (ca. 74 bis 77% Aktivsubstanzgehalt; INCI-Bezeichnung: Quaternium-27, Propylene Glycol) (Goldschmidt) | |
| ²¹ Fettalkohol-Methyltriethanolammoniummethylsulfatdialkylester-Gemisch (INCI-Bezeichnung: Distearoylethyl Hydroxyethylmonium Methosulfate, Cetearyl Alcohol) (Cognis) | |
| ²² Stearinsäure-N-(3-dimethylamino)propylamid (INCI-Bezeichnung: Stearamido-propyl Dimethylamine) (Degussa) | |
| ²³ Weizenproteinhydrolysat (mind. 20% Festkörper; INCI-Bezeichnung: Aqua (Water), Hydrolyzed Wheat Protein, Sodium Benzoate, Phenoxyethanol, Methylparaben, Propylparaben) (Cognis) | |
| ²⁴ Dimethylcyclosiloxan-Dimethylpolysiloxanol-Gemisch (ca. 13% Festkörper; INCI-Bezeichnung: Cyclomethicone, Dimethiconol) (Dow Coming) | |

Der pH-Wert des Nachbehandlungsmittel betrug 3,1.

### 4. Durchführung des erfindungsgemäßen Verfahrens

### 4.1 Färbung

Die Färbecreme A beziehungsweise B wurde auf 5cm lange Strähnen (Euro Naturhaar, weiß, Fa. Kerling) aufgetragen, und dort 30min bei 32°C belassen. Danach wurde das Haar gespült, mit einem üblichen Haarwaschmittel ausgewaschen und anschließend getrocknet.

### 4.2 Farbveränderung

Die Zusammensetzungen (Ia) beziehungsweise (Ib) wurden mit der Zusammensetzung (II) gemäß den Angaben der Tabelle 1 gemischt und auf die gefärbte Fasern (siehe Pkt. 4.1) aufgetragen. Die Einwirkzeit der farbverändernden Zubereitung betrug 30 Minuten bei 35°C. Anschließend wurden die Fasern mit dem Nachbehandlungsmittel (siehe Punkt 3) gespült und getrocknet.
Die gemessenen Farbrückstände, jeweils bezogen auf die Färbung vor der Behandlung mit dem farbverändernden Mittel, die mithilfe der Zubereitung (Ia) erhalten wurden, sind in Tabelle 2 wiedergegeben.

**Tabelle 1:**

| Farbveränderndes Mittel | Zubereitung (Ia bzw. Ib) [Gew.-%] | Zubereitung(II) [Gew.-%] | Prozentualer Anteil Natriumdithionit |
|---|---|---|---|
| E1 | 89,47 | 10,53 | 10% |
| E2 | 94,74 | 5,26 | 5% |
| E3 | 96,84 | 3,16 | 3% |
| E4 | 98,95 | 1,05 | 1% |

**Tabelle 2:**

| Farbverändemdes Mittel | Färbung | Farbrückstand in [%] |
|---|---|---|
| E1 | Färbemittel A | 13% |
| | Färbemittel B | 10% |
| E2 | Färbemittel A | 22% |
| | Färbemittel B | 28% |
| E3 | Färbemittel A | 47% |
| | Färbemittel B | 43% |
| E4 | Färbemittel A | 70% |
| | Färbemittel B | 70% |

## Patentansprüche

1. Verfahren zur Behandlung keratinischer Fasern, bei dem in einem ersten Schritt die Fasern in üblicher Weise gefärbt werden und in einem zweiten Schritt die Fasern mit einem Mittel (M), enthaltend mindestens ein Dithionitsalz und mindestens ein Tensid, behandelt werden.

2. Verfahren nach Anspruch 1, **dadurch gekennzeichnet, dass** das Mittel (M) mindestens ein anionisches Tensid enthält.

3. Verfahren nach einem der Ansprüche 1 oder 2, **dadurch gekennzeichnet, dass** der erste Schritt eine oxidative Färbung ist.

4. Verfahren nach einem der Ansprüche 1 oder 2, **dadurch gekennzeichnet, dass** der erste Schritt eine Färbung auf Basis von direktziehenden Farbstoffen ist.

5. Verfahren nach einem der Ansprüche 1 bis 4, **dadurch gekennzeichnet, dass** das Dithionitsalz Natriumdithionit ist.

6. Verfahren nach einem der Ansprüche 1 bis 5, **dadurch gekennzeichnet, dass** die Einwirkzeit der Mittels (M) zwischen 5 und 60 Minuten, insbesondere zwischen 15 und 45 Minuten beträgt.

7. Verfahren nach einem der Ansprüche 1 bis 6, **dadurch gekennzeichnet, dass** die Behandlung mit dem Mittel (M) unmittelbar nach der Färbung zur Veränderung der erzielten Nuance erfolgt.

8. Verfahren nach einem der Ansprüche 1 bis 7, **dadurch gekennzeichnet, dass** die Behandlung mit dem Mittel (M) zur Beschleunigung des Auswaschens der Färbung erfolgt.

9. Verfahren nach einem der Ansprüche 1 bis 8, **dadurch gekennzeichnet, dass** die Fasern unmittelbar nach der Applikation des Mittels (M) mit einer Folie umwickelt werden.

10. Verfahren nach einem der Ansprüche 1 bis 9, **dadurch gekennzeichnet, dass** das Mittel (M) mithilfe einer sauer eingestellten Nachspülung aus den Fasern ausgewaschen wird.

11. Mittel zur Farbveränderung gefärbter keratinischer Fasern, das unmittelbar vor der Anwendung durch Mischen einer ersten, wässrigen oder wässrig-alkoholischen Zubereitung (I) mit einer zweiten wasserfreien Zubereitung (II), enthaltend mindestens ein Dithionitsalz, erhalten wird, wobei mindestens eine der beiden Zubereitungen mindestens ein Tensid enthält.

12. Mittel nach Anspruch 11, **dadurch gekennzeichnet, dass** das Tensid in der Zubereitung (I) enthalten ist.

13. Mittel nach einem der Ansprüche 11 oder 12, **dadurch gekennzeichnet, dass** das Tensid ein anionisches Tensid ist.

14. Mittel nach einem der Ansprüche 11 bis 13, **dadurch gekennzeichnet, dass** eine der Zubereitungen mindestens eine Ölkomponente enthält.

15. Mittel nach Anspruch 14, **dadurch gekennzeichnet, dass** die Ölkomponente ein Fettalkohol ist.

16. Mittel nach einem der Ansprüche 14 oder 15, **dadurch gekennzeichnet, dass** die Ölkomponente in der Zubereitung (I) enthalten ist.

17. Mittel nach einem der Ansprüche 11 bis 16, **dadurch gekennzeichnet, dass** es ein weiteres Reduktionsmittel enthält.

18. Mittel nach Anspruch 17, **dadurch gekennzeichnet, dass** das weitere Reduktionsmittel ein Disulfitsalz ist.

19. Mittel nach einem der Ansprüche 17 oder 18, **dadurch gekennzeichnet, dass** das weitere Reduktionsmittel in der Zubereitung (II) enthalten ist.

20. Verwendung einer Anwendungszubereitung, enthaltend mindestens ein Dithionitsalz und mindestens ein Tensid, zur Beschleunigung des Auswaschens von Färbungen keratinischer Fasern.

21. Verwendung einer Anwendungszubereitung, enthaltend mindestens ein Dithionitsalz und mindestens ein Tensid, zur Nachnuancierung von Färbungen keratinischer Fasern.

22. Verwendung gemäß einem der Ansprüche 20 oder 21, **dadurch gekennzeichnet, dass** die Anwendungszubereitung mindestens ein anionisches Tensid enthält.
